(19) Europäisches Patentamt
European Patent Office
Office européen des brevets

(11) Numéro de publication: **0 046 126 B1**

(12) **FASCICULE DE BREVET EUROPEEN**

(45) Date de publication du fascicule du brevet: **18.12.85**

(51) Int. Cl.⁴: **A 61 M 1/14**

(21) Numéro de dépôt: **81420097.8**

(22) Date de dépôt: **29.06.81**

(54) Rein artificiel - régulation de la pression du liquide de dialyse.

(30) Priorité: **01.08.80 FR 8017326**

(43) Date de publication de la demande:
**17.02.82 Bulletin 82/07**

(45) Mention de la délivrance du brevet:
**18.12.85 Bulletin 85/51**

(84) Etats contractants désignés:
**BE CH DE GB IT LI NL SE**

(56) Documents cités:
**FR-A-2 163 906**
**FR-A-2 288 529**
**FR-A-2 339 406**

**The Lancet, 11 Février 1961, page 340-341**

(73) Titulaire: **HOSPAL INDUSTRIE**
**7, Avenue Lionel Terray**
**F-69330 Meyzieu (FR)**

(72) Inventeur: **Vantard, Georges**
**20, rue des prés de Noisy**
**F-93460-Gournay Sur Marne (FR)**

(74) Mandataire: **Gauckler, Jacques et al**
**RHONE-POULENC RECHERCHES HOSPAL-**
**BREVETS Centre de Recherches de Saint-Fons**
**B.P. 62**
**F-69190 Saint-Fons (FR)**

Courier Press, Leamington Spa, England.

## Description

La présente invention à la réalisation de laquelle a collaboré Monsieur Georges VANTARD, concerne un rein artificiel. Elle concerne plus particulièrement un dispositif de régulation automatique de la pression du liquide de dialyse en fonction des quantités d'ultrafiltrat que l'on veut retirer au sang d'un patient selon le traitement particulier prévu à chaque séance d'hémodialyse.

Le Certificat d'addition français n° 2.339.406 décrit un rein artificiel comportant divers moyens de régulation et de contrôle de la pression et du débit du liquide de dialyse dans l'hémodialyseur. Cependant, le circuit de liquide de dialyse qui n'est pas bouclé sur lui-même et qui est isolé de l'atmosphère, ne comporte pas de moyen permettant de prélever au patient des quantités prédéterminées et mesurables d'ultrafiltrat.

Dans la revue "The Lancet" publiée le 11 Février 1961, pages 340—341, est décrit un rein artificiel comportant un circuit bouclé sur lui-même et ouvert à l'atmosphère qui permet de recueillir par trop-plein des quantités de liquide de dialyse égales aux quantités d'ultrafiltrat ayant traversé la membrane du dialyseur. Toutefois, ce dispositif ne permet pas d'imposer à l'avance les quantités d'ultrafiltrat que l'on désire retirer au patient.

Le FR—A—2.163.906 décrit un rein artificiel comportant un circuit bouclé sur lui-même, isolé de l'atmosphère et des moyens pour imposer la quantité prévue d'ultrafiltrat qui doit être retirée à un patient lors de chaque traitement. Cependant la mise en oeuvre de ce rein artificiel peut nécessiter des moyens assez importants en matériel ou en personnel qualifié.

Un objet de la présente invention est un rein artificiel qui permette d'imposer les quantités prévues d'ultrafiltrat qui doivent être retirées à un patient lors de chaque traitement, mais dont la construction soit plus simple et plus économique et dont le fonctionnement soit encore plus fiable.

Un autre objet de la présente invention est un rein artificiel qui permette une régulation de la pression du liquide de dialyse dans l'hémodialyseur sans intervention extérieure, en fonction directe de l'ultrafiltrat retiré au patient.

D'autres objets de la présente invention apparaîtront au cours de la description qui va suivre.

Il a maintenant été trouvé un rein artificiel comprenant un hémodialyseur relié à un circuit pour le liquide de dialyse, de volume interne pratiquement contstant, bouclé sur lui-même avec ou sans recirculation du liquide, comprenant aussi une pompe de circulation, ainsi que des moyens pour soutirer hors dudit circuit et des moyens pour mesurer des quantités de liquide égales aux quantités d'ultrafiltrat que l'on désire prélever dans le sang du patient à travers la membrane de l'hémodialyseur. Ce rein artificiel est caractérisé en ce que le circuit est ouvert à l'atmosphère, en ce qu'il comprend, en aval de l'hémodialyseur, une chambre et une résistance hydraulique variable et en ce que ladite résistance est commandée par le niveau du liquide dans ladite chambre qu'elle tend à maintenir constant afin de réguler automatiquement la pression transmembranaire dans l'hémodialyseur en fonction des quantités d'ultrafiltrat prélevées au patient.

La compréhension de l'invention sera facilitée par les figures ci-jointes qui représentent schématiquement et sans échelle déterminée divers exemples de réalisation.

La figure 1 est le schéma d'un premier mode de réalisation préféré d'un rein artificiel selon l'invention.

La figure 2 est le schéma d'un second mode de réalisation d'un rein artificiel selon l'invention.

La figure 3 est le schéma d'un troisième mode de réalisation d'un rein artificiel selon l'invention.

La figure 4 est le schéma d'un quatrième mode de réalisation d'un rein artificiel selon l'invention.

Afin de faciliter la compréhension de l'invention, les éléments homologues dans les différentes figures portent les mêmes numéros.

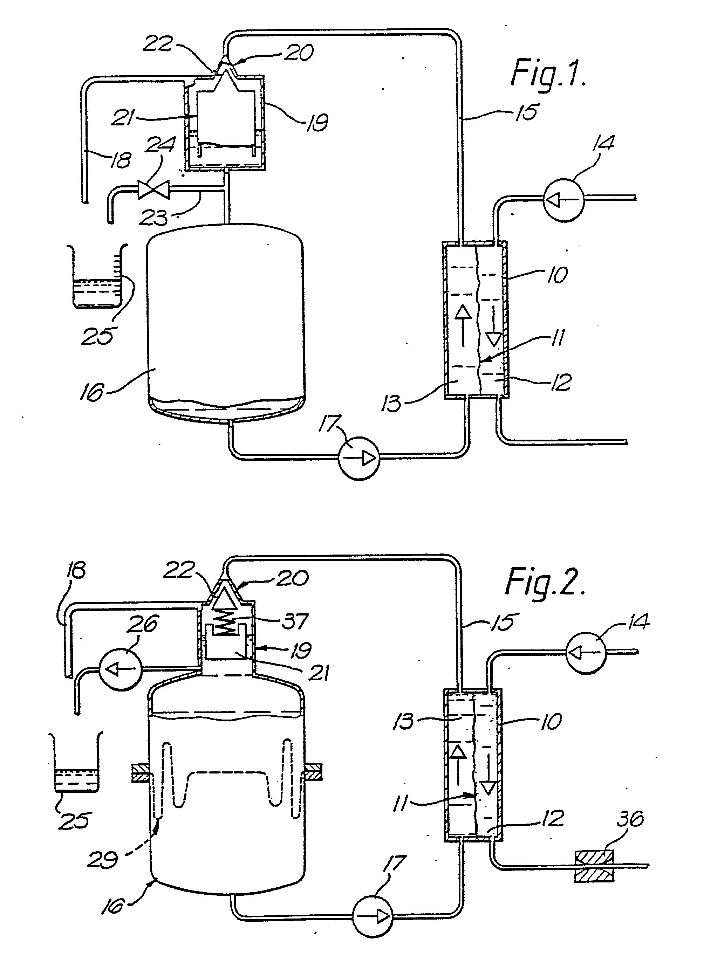

En se référant à la figure 1, le rein artificiel selon l'invention comporte un hémodialyseur (10) divisé en deux compartiments (12 et 13) par une membrane (11) permettant simultanément la dialyse et l'ultrafiltration du sang. Le compartiment (12) est parcouru par le sang à traiter, refoulé par la pompe de circulation (14). Le liquide de dialyse traverse le compartiment (13), de préférence à contre courant du sang, comme indiqué par les flèches.

Le circuit de liquide de dialyse (15) est bouclé sur lui-même; il comporte un réservoir (16) dont le volume n'est pas critique, ainsi que des moyens de chauffage conventionnels du liquide de dialyse (non représentés). Le liquide de dialyse se déplace sous l'action de la pompe de circulation (17) située à l'amont de l'hémodialyseur, c'est-à-dire reliée de préférence directement à l'orifice d'entrée du liquide de dialyse dans l'hémodialyseur. Ce circuit bouclé, constitué par le réservoir (16) et par les éléments le reliant à l'hémodialyseur (10), a un volume interne pratiquement constant, c'est-à-dire qu'il conserve un volume interne pratiquement constant, quelles que soient les contraintes auxquelles il peut être normalement soumis. Comme ce circuit est ouvert à l'atmosphère, grâce au conduit (18), ces contraintes sont généralement faibles et sa construction est donc économique.

Selon l'invention, ce circuit comprend une résistance hydraulique variable située à l'aval de l'hémodialyseur, c'est-à-dire reliée de préférence directement à l'orifice de sortie du liquide de dialyse de l'hémodialyseur. Par résistance hydraulique variable, on entend tout moyen mécanique connu permettant de régler, au moyen d'une perte de charge locale, la pression d'un fluide à une valeur désirée. Cette résistance hydraulique variable est constituée figure 1 par un obturateur dont la partie mobile est solidaire d'un flotteur. A cet effet une chambre (19) est munie à sa partie supérieure d'un orifice (20) pour l'introduction du liquide de dialyse. Cet orifice, d'axe vertical, est de préférence évasé vers le bas, les parois formant un cône vertical.

Un flotteur (21), surmonté d'un pointeau (22) de préférence conique, coopère avec l'orifice évasé (20), de manière connue en soi, de façon à constituer un dispositif obturateur capable d'assurer une fonction de régulation automatique de pression du liquide de dialyse. Poussé par le flotteur, le pointeau (22), peut obturer totalement l'orifice (20). Dès que le flotteur amorce une descente, il se dégage et libère partiellement l'orifice (20). La résistance hydraulique variable est ainsi asservie au maintien d'un niveau sensiblement constant de liquide de dialyse dans la chambre (19) et la pression du liquide de dialyse à l'amont, notamment dans l'hémodialyseur, est ainsi automatiquement régulée.

Selon un mode de réalisation préféré, la résistance hydraulique variable reçoit du liquide de dialyse à la fois l'information pour sa commande et l'énergie motrice pour son fonctionnement. Ainsi la pression du liquide de dialyse dans l'hémodialyseur est régulée automatiquement sans l'intervention d'un circuit hydraulique auxiliaire, ni d'aucun moyen mécanique annexe, ni d'un circuit électrique ou électronique. D'où simplicité, économie et fiabilité, tant dans la construction que dans le fonctionnement du rein artificiel selon l'invention.

En outre un conduit latéral (23), muni de moyens de réglage de débit, par exemple une vanne de réglage (24), est raccordé au circuit bouclé (15) en un point situé généralement entre la chambre (19) et le réservoir (16), et de préférence juste en-dessous de la ligne le long de laquelle s'établit le niveau de liquide dans la chambre (19).

Un récipient gradué (25) permet de mesurer à chaque instant le volume de liquide recueilli qui généralement ne dépasse pas quelques litres.

Le fonctionnement du rein artificiel représenté figure 1 est le suivant. On introduit préalablement le liquide de dialyse dans le circuit (15) que l'on remplit, par exemple à l'aide du conduit (23) et de la pompe (17). Un niveau sensiblement constant peut s'établir dans la chambre (19) lorsque, le flotteur (21) étant soulevé par le liquide de dialyse, le pointeau (22) s'engage à l'intérieur de l'orifice évasé (20), limitant ainsi la section de passage du liquide de dialyse.

On fait alors circuler le sang dans l'hémodialyseur où, principalement sous l'effet de la pompe (14) et des pertes de charge à l'aval de la pompe, en particulier de la fistule de réinjection du sang chez le patient, il exerce sur la membrane une pression moyenne constamment supérieure à la pression atmosphérique, pression que l'on peut maintenir aisément à des valeurs supérieures ou à la rigueur égales à la pression du liquide de dialyse dans l'hémodialyseur, compte-tenu de la pression osmotique du sang, dite pression oncotique.

Le circuit (15) étant rempli, on ferme la vanne (24) et l'on suppose par exemple que le pointeau (22) laisse le liquide de dialyse s'écouler librement par l'orifice (20) dans la chambre (19). La pression atmosphérique s'exerçant sur le niveau du liquide de dialyse dans la chambre (19), la pression moyenne du liquide de dialyse dans l'hémodialyseur s'établit à une valeur minimum supérieure à la pression atmosphérique et que l'on sait maintenir inférieure à celle du sang. Il en résulte entre les compartiments (12 et 13) de l'hémodialyseur une différence de pression moyenne connue sous le nom de pression transmembranaire qui a pour effet de créer à travers la membrane un flux d'ultrafiltrat s'écoulant du sang vers le liquide de dialyse.

Ce flux augmente le volume de liquide contenu dans le circuit (15), de volume interne pratiquement constant. Il élève le niveau du liquide dans la chambre (19), donc le niveau du flotteur (21), ce qui conduit à la fermeture progressive de l'obturateur (20, 22) jusqu'à ce que, la pression du liquide de dialyse augmentant régulièrement, elle atteigne une valeur pour laquelle le flux d'ultrafiltrat devient nul.

Le flotteur reste alors à niveau constant: un équilibre est établi. Si, au cours du traitement, la pression sanguine subit diverses fluctuations, le dispositif d'obturation régule à tout instant automatiquement la pression du liquide de dialyse pour maintenir constamment le flux d'ultrafiltrat à une valeur nulle.

Supposons que l'on décide maintenant de prélever une quantité déterminée d'ultrafiltrat au patient: il suffit d'ouvrir la vanne (24) puis de soutirer et de mesurer dans le récipient gradué (25) une quantité de liquide égale à la quantité d'ultrafiltrat que l'on désire prélever.

En effet on prélèvera à volonté, soit un volume, soit un débit déterminé d'ultrafiltrat en fixant le taux et la durée d'ouverture de la vanne (24). Ces conditions d'ouverture de la vanne (24) peuvent permettre soit un prélèvement immédiat, soit un prélèvement à débit constant, soit encore un prélèvement selon un débit variable, conforme à un programme approprié choisi par le responsable du traitement en fonction de l'état et du comportement du patient, à l'aide de tout dispositif connu en soi qui asservit l'ouverture de la vanne en fonction de ce programme.

Dès l'ouverture de la vanne (24) le flotteur descend, libérant l'obturateur, ce qui fait chuter la pression du liquide de dialyse, notamment dans l'hémodialyseur. Sous l'effet de la pression transmembranaire ainsi obtenue, le sang ultrafiltre à travers la membrane. La quantité d'ultrafiltrat recueillie dans le circuit (15) fait remonter progressivement le niveau du liquide de dialyse dans la chambre (19), donc le flotteur, ce qui tend à refermer progressivement l'obturateur. Ce mouvement se poursuit jusqu'à ce que la quantité d'ultrafiltrat compense exactement la quantité de liquide soutirée hors du circuit (15) et recueillie dans le récipient gradué (25).

Le dispositif décrit a donc pour effet de réguler instantanément, directement et automatiquement la pression transmembranaire, donc la quantité (volume ou débit) d'ultrafiltrat à travers la membrane pour la maintenir constamment égale à la quantité (volume ou débit) de liquide prélevé volontairement hors du circuit de liquide de dialyse (15).

Ce rein artificiel présente l'avantage de permettre un fonctionnement, comme on l'a vu, avec un taux d'ultrafiltration nul. Il comporte en outre ses propres sécurités. Ainsi, si pour une raison quelconque, par exemple une fausse manoeuvre lors du remplissage, le niveau du liquide de dialyse dans le circuit (15) monte au-dessus d'une valeur normale, tout ou partie de l'excédent peut s'écouler par le conduit (18) fonctionnant le cas échéant en siphon. D'une part l'excès de liquide peut être recueilli dans le récipient (25), il est donc mesurable et l'on peut en tenir compte dans le bilan du traitement. D'autre part aucune pression dangereuse ne risque ainsi de s'exercer sur la membrane ou sur le sang.

Inversement si du liquide s'écoule brusquement par le conduit (23), seul le volume de liquide au-dessus du niveau de la tubulure (23) s'échapperait par gravité et il est aisé de limiter automatiquement ce volume ou ce débit, tant à la construction que lors de l'utilisation du rein artificiel selon l'invention.

En outre ce système est particulièrement économique. Le volume de la chambre (19) n'est pas critique et elle n'est représentée sur la figure à une échelle différente des autres éléments du circuit que pour une plus grande clarté. En fait les différents éléments du circuit bouclé peuvent avantageusement être conçus à usage unique afin d'éviter tout traitement préalable de stérilisation. Dans ce but, on préfère revêtir intérieurement le réservoir (16) de volume constant d'une poche un matière plastique souple, imperméable, amovible que l'on raccorde aux orifices d'entrée et/ou de sortie de ce réservoir. Ainsi, après chaque séance, seule la poche est jetée, la réservoir lui-même peut être réutilisé sans avoir à être stérilisé.

La présente invention peut faire l'objet de différents modes de réalisation. A titre d'exemples les figures 2, 3 et 4 représentent certains de ces modes de réalisation. Naturellement on ne sort pas du cadre de la présente invention si l'on combine entre elles des dispositions particulières représentées sur des figures différentes.

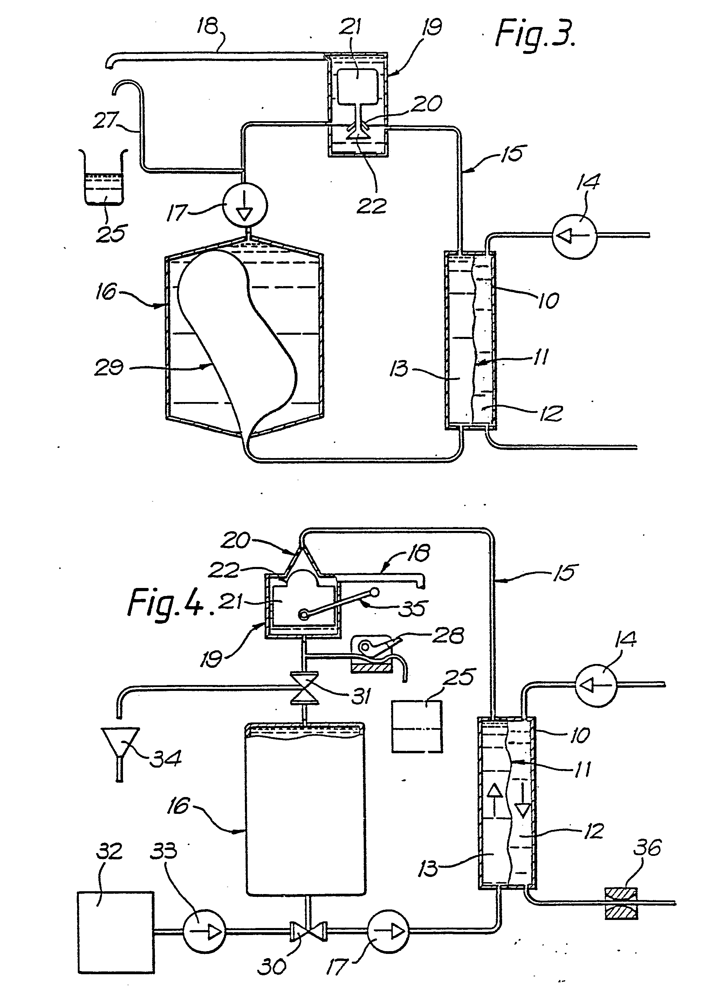

Le dispositif régulateur avec obturateur à flotteur peut être constitué par tous moyens connus. Ainsi à titre d'exemples la chambre (19), figure 2, est incorporée au réservoir (16), le flotteur (21) peut prendre différentes formes, de révolution ou non, être ouvert à la partie inférieure en forme de cloche [figure 1] ou constituer un volume fermé [figures 2, 3 et 4] creux ou plein, il peut être alors constitué par un matériau expansé. Le guidage du flotteur peut être assuré par tous moyens connus, par exemple par les parois internes de la chambre (19) ou par un levier articulé (35) [figure 4]. Le pointeau est préférentiellement conique, mais il peut avoir toute autre forme appropriée, par exemple hémisphérique [figure 4].

Le pointeau obturateur peut être incorporé au flotteur [figures 1 et 4]. Il peut être relié de manière rigide au flotteur [figure 3] et ainsi être solidaire du flotteur. Il peut également être relié de manière élastique au flotteur [figure 2], par exemple par l'intermédiaire d'un ressort qui peut avoir une action démultiplicatrice entre le niveau du liquide de dialyse dans la chambre (19) et la pression du liquide de dialyse, notamment dans l'hémodialyseur. Le dispositif obturateur peut être situé soit au-dessus du flotteur [figures 1, 2 et 4], soit au-dessous [figure 3]. On peut utiliser un ou plusieurs flotteurs et/ou dispositifs obturateurs. Par exemple on peut disposer en parallèle deux ensembles distincts flotteurs-obturateurs (non représentés), de dimensions différentes, fonctionnant à obturation décalée, la régulation la plus fine étant ainsi assurée seulement par le plus petit ensemble. Les moyens de soutirage de liquide hors du circuit bouclé de liquide de dialyse peuvent être constitués soit par une vanne de réglage (24) [figure 1], soit par un rétreint réglable [clamp] (28) [figure 4], soit par un simple trop plien de hauteur réglable (27) [figure 3], soit préférentiellement par une pompe (26) [figure 2], occlusive, qui peut imposer l'extraction de quantités prédéterminées de liquide quel que soit notamment le degré de colmatage de la membrane. A cette pompe peut être associée un volucomteur de tout type connu (non représenté) mesurant, totalisant et/ou enregistrant avec précision le volume de liquide débité. On remarque que le dispositif selon l'invention permet à la pompe (26) d'extraire sans le moindre risque du liquide de dialyse à un débit quelconque, même supérieur au débit de l'ultrafiltrat à travers la membrane (11).

La pompe (17) de circulation de liquide de dialyse est avantageusement disposée à l'amont de l'hémodialyseur (10), préférentiellement entre le réservoir (16) et l'hémodialyseur (10). Elle peut aussi [cf. figure 3] être disposée entre la chambre (19) et le réservoir (16), poussant le liquide de dialyse usagé dans le réservoir (16) et déplaçant ainsi, à la même pression à travers un diaphragme souple (29), une même quantité de liquide de dialyse frais à travers l'hémodialyseur (10).

Le réservoir (16) a un volume constant; il est préférentiellement cylindrique et muni de fonds coniques [figure 3] ou semi-elliptiques [figures 1 et 2]. Selon la figure 1, le liquide de dialyse peut être recyclé dans l'hémodialyseur jusqu'à la fin du traitement. Le cas échéant, le liquide de dialyse peut traverser un filtre épurateur, par exemple garni de charbon actif, non representé.

Toutefois on préfère opérer en "single pass", c'est-à-dire ne faire passer qu'une seule fois le liquide de dialyse dans l'hémodialyseur. Un diaphragme souple (29) [figures 2 et 3] sépare de manière étanche le liquide de dialyse frais du liquide usagé.

On peut aussi ne mettre en oeuvre qu'un réservoir (16) de volume trés réduit si, comme représenté figure 4 on relie simultanément et périodiquement le réservoir (16) par deux vannes trois voies (30 et 31) d'une part à une source de liquide de dialyse frais (32) à l'aide d'une pompe (33) et d'autre part à l'égout (34). Un diaphragme souple et étanche (non représenté) peut maintenir le cas échéant constamment séparés le liquide de dialyse frais du liquide de dialyse usagé.

Le fonctionnement de l'installation représentée figure 4 est alors le suivant. Les robinets trois voies permettent au liquide de dialyse de circuler normalement dans le circuit bouclé (15) grâce à la pompe (17) jusqu'à ce que le liquide usagé doive être remplacé par du liquide frais. Périodiquement donc, les vannes trois voies (30 et 31) isolent simultanément le reste du circuit bouclé de liquide de dialyse et permettent le remplacement du liquide de dialyse usagé par un volume égal de liquide de dialyse frais qui est soumis à un nouveau cycle. Si désiré, une canalisation de by-pass (non représentée) peut relier la chambre (19) à l'orifice d'aspiration de la pompe (17), afin de ne pas interrompre la circulation du liquide de dialyse pendant le remplacement du liquide de dialyse usagé dans le réservoir (16).

Le cas échéant un dispositif de fermeture partielle du circuit sang tel qu'un clamp (36) [figures 2 et 4] peut être disposé en aval de l'hémodialyseur afin d'augmenter si nécessaire la pression sang dans l'hémodialyseur pour éviter qu'elle ne soit inférieure à la pression du liquide de dialyse. On a ainsi toujours la possibilité de maintenir une pression transmembranaire positive ou nulle permettant le traitement du sang par dialyse et ultrafiltration simultanées. Eventuellement la commande de fermeture du clamp peut être exécutée ou contrôlée par tout dispositif connu en soi, comparant la quantité de liquide recueillie dans le récipient (25) à la quantité d'ultrafiltrat que l'on désire retirer.

Lorsque la membrane (11) est du type à haut flux, c'est-à-dire ayant une perméabilité à l'eau supérieure ou égale à 15 ml/heure $\times$ m$^2$ $\times$ mmHg, un taux d'ultrafiltration suffisant est obtenu pour une pression transmembranaire relativement faible et, dans ces conditions, l'emploi du clamp (36) n'est généralement pas nécessaire.

On peut naturellement aussi combiner le rein artificiel selon l'invention à un dispositif de traitement du sang par plasmaphérèse de tout type connu en soi.

### Revendications

1. Rein artificiel comprenant un hémodialyseur (10) relié à un circuit (15) pour le liquide de dialyse, de volume interne pratiquement constant, bouclé sur lui-même avec ou sans recirculation du liquide, comprenant aussi une pompe de circulation (17), ainsi que des moyens (24, 26, 27 ou 28) pour soutirer hors dudit circuit et des moyens (25) pour mesurer des quantités de liquide égales aux quantités d'ultrafiltrat que l'on désire prélever dans le sang du patient à travers la membrane (11) de l'hémodialyseur (10), caractérisé en ce que le circuit (15) est ouvert à l'atmosphère, en ce qu'il comprend, en aval de l'hémodialyseur (10), une chambre (19) et une résistance hydraulique variable (20, 22) et en ce que ladite résistance est commandée par le niveau du liquide dans ladite chambre (19) qu'elle tend à maintenir constant afin de réguler automatiquement la pression transmembranaire dans l'hémodialyseur (10) en fonction des quantités d'ultrafiltrat prélevées au patient.

2. Rein artificiel selon la revendication 1, caractérisé en ce que ladite résistance hydraulique variable (20, 22) reçoit du liquide de dialyse à la fois l'information pour sa commande et l'énergie motrice pour son fonctionnement.

3. Rein artificiel selon l'une des revendications 1 ou 2, caractérisé en ce que ladite résistance hydraulique variable est constituée par un obturateur (20, 22) dont la partie mobile (22) est reliée à un flotteur (21).

4. Rein artificiel selon la revendication 3, caractérisé en ce que la partie mobile (22) de l'obturateur est relié de façon rigide au flotteur (21).

5. Rein artificiel selon la revendication 3, caractérisé en ce que la partie mobile (22) de l'obturateur est reliée de façon élastique au flotteur (21).

6. Rein artificiel selon l'une quelconque des revendications précédentes, caractérisé en ce qu'un diaphragme (29) étanche et souple sépare le liquide de dialyse usagé du liquide de dialyse frais.

7. Rein artificiel selon l'une quelconque des revendications précédentes, caractérisé en ce que ledit circuit de dialyse comporte des moyens (30, 31) pour périodiquement remplacer le liquide de dialyse usagé par du liquide de dialyse frais, lesdits moyens permettant de commuter simultanément le réservoir (16) sur une source de liquide de dialyse frais (32) et l'égout (34).

8. Rein artificiel selon l'une quelconque des revendications précédentes, caractérisé en ce que tous les éléments constituant ledit circuit bouclé de liquide de dialyse et en contact avec ledit liquide sont à usage unique, ledit réservoir (16) étant garni intérieurement d'au moins une poche souple imperméable.

### Patentansprüche

1. Künstliche Niere mit einem Hämodialysator, der mit einem Kreislauf (15) für die Dialyseflüssigkeit mit praktisch konstantem Innenvolumen verbunden ist, der geschlossen ist mit oder ohne Rezirkulation der Dialyseflüssigkeit, der eine Umwälzpumpe (17) und eine Einrichtung (24, 26, 27 oder 28) für das Abziehen und eine Einrichtung (25) für das Messen von Flüssigkeitsmengen aufweist, die gleich den Ultrafiltratmengen sind, die dem Blut eines Patienten durch die Membran (11) des Hämodialysators (10) hindurch entnommen werden sollen, dadurch gekennzeichnet, daß der Kreislauf (15) zur Atmosphäre offen ist, daß er stromab des Hämodialysators (10) eine Kammer (19) und einen veränderlichen hydraulischen Widerstand (20, 22) aufweist, und daß der Widerstand durch das Niveau der Flüssigkeit in der Kammer (19) gesteuert wird, das er konstant zu halten bestrebt ist, um automatisch den Transmembrandruck im Hämodialysator (10) in Abhängigkeit von dem Patienten entnommenem Ultrafiltratmengen zu regeln.

2. Künstliche Niere nach Anspruch 1, dadurch gekennzeichnet, daß der Widerstand (20, 22) mit der Dialyseflüssigkeit gleichzeitig die Information zu seiner Steuerung und die Antriebsenergie für seinen Betrieb empfängt.

3. Künstliche Niere nach Anspruch 1 oder 2, dadurch gekennzeichnet, daß der Widerstand (20, 22) aus einem Verschluß besteht, dessen beweglicher Teil (22) mit einem Schwimmer (21) verbunden ist.

4. Künstliche Niere nach Anspruch 3, dadurch gekennzeichnet, daß der bewegliche Teil mit dem Schwimmer (21) starr verbunden ist.

5. Künstliche Niere nach Anspruch 3, dadurch gekennzeichnet, daß der bewegliche Teil (22) mit dem Schwimmer (21) elastisch verbunden ist.

6. Künstliche Niere nach einem der vorhergehenden Ansprüche, dadurch gekennzeichnet, daß eine dichte und biegsame Membran (21) die verbrauchte Dialyseflüssigkeit von der frischen Dialyseflüssigkeit trennt.

7. Künstliche Niere nach einem der vorhergehenden Ansprüche, dadurch gekennzeichnet, daß der Dialysekreislauf eine Einrichtung (30, 31) aufweist zum periodischen Ersetzen der verbrauchten Dialyseflüssigkeit durch frische Dialyseflüssigkeit, wobei diese Einrichtung (30, 31) den Vorratsbehälter (16) gleichzeitig auf eine Quelle (32) für frische Dialyseflüssigkeit und auf einen Ablauf (34) umschaltet.

8. Künstliche Niere nach einem der vorhergehenden Ansprüche, dadurch gekennzeichnet, daß alle den geschlossenen Dialyseflüssigkeitskreislauf bildenden und mit der Dialyseflüssigkeit in Verbindung stehenden Elemente für einen Einmalgebrauch bestimmt sind, wobei der Vorratsbehälter (16), innen mit wenigstens einem biegsamen, undurchlässigen Beutel bedeckt ist.

## Claims

1. An artificial kidney comprising a haemodialyser (10) connected to a circuit (15) for the dialysis liquid with a practically constant internal volume, forming a loop in itself with, or without recirculation of the liquid, also comprising a circulating pump (17) as well as means (24, 26, 27 or 28) for drawing off out of the said circuit and means (25) for measuring quantities of liquid equal to the quantities of ultrafiltrate which it is desired to remove from the patient's blood through membrane (11) of the haemodialyser (10), characterised in that circuit (15) is open to the atmosphere, in that it comprises, downline from haemodialyser (10), a chamber (19) and a variable hydraulic resistor (20, 22) and in that the said resistor is controlled by the level of liquid in the said chamber (19) which it tends to keep constant so as to regulate automatically the trans-membrane pressure in the haemodialyser (10) depending on the ultrafiltrate quantities taken from the patient.

2. An artificial kidney according to Claim 1, characterised in that the said variable hydraulic resistor (20), (22) receives, from the dialysis liquid, both the information for its control and the driving energy for its operation.

3. An artificial kidney according to one of Claims 1 or 2, characterised in the said variable hydraulic resistor is constituted by an obturator (20), (22) whose movable part (22) is joined to a float (21).

4. An artificial kidney according to Claim 3, characterised in that the movable part (22) of the obturator is rigidly joined to float (21).

5. An artificial kidney according to Claim 3, characterised in that the movable part (22) of the obturator is elastically joined to float (21).

6. An artificial kidney according to any one of the preceding Claims, characterised in that a leakproof and pliable diaphragm (29) separates the used dialysis liquid from the fresh dialysis liquid.

7. An artificial kidney according to any of the preceding Claims, characterised in that the said dialysis circuit comprises means (30, 31) for periodically replacing the used dialysis liquid by fresh dialysis liquid, the said means allowing reservoir (16) to be simultaneously connected to a source of fresh dialysis liquid (32) and the waste outlet (34).

8. An artificial kidney according to any one of the preceding Claims, characterised in that all the elements which constitute the said looped dialysis liquid circuit and are in contact with the said liquid, are to be used once only, the said reservoir (16) being lined internally with at least one pliable impermeable bag.

Fig.1.

Fig.2.

Fig.3.

Fig.4.